# EUROPEAN PATENT APPLICATION

(11) **EP 1 629 770 A1**
(43) Date of publication of application: **01.03.2006**
(21) Application number: 05018416.7
(22) Date of filing: 24.08.2005
(51) Int. Cl.: A61B 5/0404

(54) **Portable electrocardiograph**

(30) Priority: 27.08.2004 JP 2004248012
(71) Applicant: Omron Healthcare Co., Ltd., Kyoto 615-0084 (JP)
(72) Inventor: Moroki, Yoko c/o Omron Healthcare Co., Ltd., Kyoto-shi Kyoto 615-0084 (JP); Yamamoto, Norihito c/o Omron Healthcare Co., Ltd., Kyoto-shi Kyoto 615-0084 (JP); Ishida, Junichi c/o Omron Healthcare Co., Ltd., Kyoto-shi Kyoto 615-0084 (JP); Tanabe, Kazuhisa c/o Omron Healthcare Co., Ltd., Kyoto-shi Kyoto 615-0084 (JP); Umeda, Masahiro c/o Omron Healthcare Co., Ltd., Kyoto-shi Kyoto 615-0084 (JP)
(74) Representative: Kilian, Helmut

(57) **Abstract**

In a portable electrocardiograph, combinations of analysis results corresponding to analysis items of an electrocardiographic waveform based on electrocardiography and display contents are stored in advance in association with each other. Thereby, the portable electrocardiograph analyzes obtained electrocardiographic waveform data to classify the electrocardiographic waveform data by the analysis items based on electrocardiography (S104). It collates a classification result being classified and the combinations of analysis results stored to select a corresponding display content (S108). It displays the selected display content as an evaluation of the electrocardiographic waveform data (S110).

## Description

### BACKGROUND OF THE INVENTION

### Field of the Invention

The present invention relates to a portable electrocardiograph, and particularly, to a portable electrocardiograph having an automatic analyzing function of an electrocardiographic wave.

### Description of the Background Art

The analysis of an electrocardiographic wave is a difficult task even for highly-skilled medical specialists. Accordingly, numerous automatic analysis technique of an electrocardiographic wave for their support have been proposed.

For example, Prior Art Document 1: Japanese Patent Laying-Open No. 04-064337 proposes an automatic electrocardiogram analyzer including: an analysis portion generating electrocardiogram analysis information and a diagnosis result based on the information; a text storage portion storing text information describing in detail the diagnosis result based on the electrocardiogram analysis information; means for outputting the diagnosis result; and means for reading and outputting text information describing in detail the diagnosis result based on the electrocardiogram analysis information. Prior Art Document 2: Japanese Patent Laying-Open No. 09-201344 proposes an electrocardiogram analysis device including: analysis means for measuring and analyzing a plurality of waveforms of heartbeats; storage means for storing diagnostic terms corresponding to results of the analysis by the analysis means; and display means for displaying waveforms and the diagnostic terms corresponding to the waveforms, wherein the analysis means automatically selects a plurality of representative heartbeats from the plurality of heartbeats with one analysis operation, successively analyzes the selected plurality of representative heartbeats, and displays diagnostic terms respectively corresponding to results of the analysis of the plurality of representative heartbeats. Both of them are technique for supporting medical specialists in analyzing an electrocardiogram, and the contents displayed as a result of analysis is premised on the use by medical specialists.

On the other hand, there is a "portable electrocardiograph" to be constantly carried by a subject having a concern about his/her heart and to be used to detect an electrocardiographic waveform when the subject observes the symptoms. In such a portable electrocardiograph also, since the electrocardiographic waveform that is a result of measurement is to be used for a diagnosis by medical specialists, it has been necessary for the subject carrying the device to have a medical specialist's diagnosis to know what is meant by the result of measurement. As a technique for solving such a problem, Prior Art Document 3: Japanese Patent Laying-Open No. 2001-046350 discloses a portable electrocardiographic monitor, wherein electrocardiographic waveform data obtained at measurement and standard data of a healthy state are compared. When a difference between them exceeds an allowable range, an abnormality is determined and an alarm is issued.

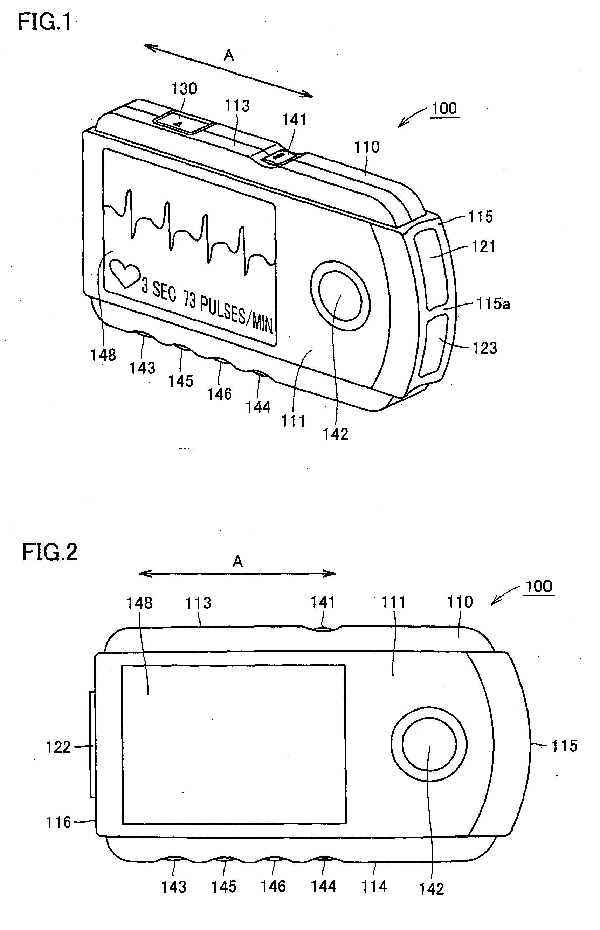

Further, such an electrocardiographic waveform automatic analysis technique is disclosed, for example, in Prior Art Document 4: Mutsuo Kaneko, Fukuda Denshi Co., Ltd., "Holter electrocardiograph", Medical Care and Computer, vol. 4, No. 5, February 1992, pp.396-402. Prior Art Document 4 describes about a Holter electrocardiographic system. With the Holter electrocardiographic system, an electrocardiographic waveform recorded by a small recorder carried by a patient is reproduced and analyzed by a reproducing device (see Fig. 1 of Prior Art Document 4).

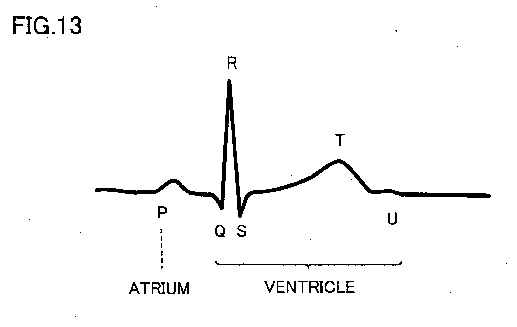

Now, referring to Fig. 13, a typical example of an electrocardiogram is described. An electrocardiogram is constituted by P, QRS, and T waves. P wave represents excitation of atrium, QRS wave represents excitation of ventricle, and T wave represents a charging period of ventricle.

The automatic analysis by a reproducing device as disclosed in the aforementioned Prior Art Document 4 is performed in such a flow shown in Fig. 2 of Prior Art Document 4. Specifically, the following process is performed. First, a portion where an artifact is detected is removed from the electrocardiogram. Next, QRS is detected, and some simple parameters indicative of features of the detected QRS are measured to perform determination. As for the parameters used for the determination, for example, R-R interval, QRS width, QRS area, QRS height, deviation (ratio of upward direction and downward direction) and the like may be included.

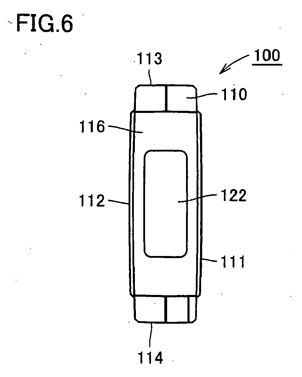

The determination is performed by relative comparison between the parameters of detected QRS and reference values. The reference values are determined in advance as parameter values associated with a normal heartbeat of the subject, based on the clustering method, for example. For example, parameters of QRS are measured and compared to an existing group. If the parameter values shows approximate values, this QRS is registered as the same group. Otherwise, a new group is formed with that QRS. Thus, QRS with approximate parameter values are gathered and grouped (see Fig. 6 of Prior Art Document 4). Then, when QRS registered as one group reaches a certain number, the parameter values of that group are checked. If they are in a general normal range, they are determined as reference values (normal values) of the subject.

When the determination of QRS ends, the level of ST portion, the shape of P and T waves and the like are also relatively compared to reference values. From total determination of them, a variety of arrhythmia are detected.

Even if the techniques related to the automatic analysis of an electrocardiographic waveform disclosed in Prior Art Documents 1, 2 and 4 are applied to the portable electrocardiograph, it is difficult for a subject not having thorough medical knowledge to grasp the contents of a result of analysis that is premised on use by medical specialists. Accordingly, even when an abnormal waveform indicates that a doctor's diagnosis is urgently required, it is difficult for the subject himself/herself to determine how to address the situation.

Further, in the portable electrocardiographic monitor disclosed in Prior Art Document 3, although an alert is issued if a difference between the electrocardiographic waveform at measurement and the standard waveform data exceeds an allowable range, the subject is not capable of knowing the contents of a result of analysis. Additionally, a method of determining a difference between the data is not clearly disclosed.

### SUMMARY OF THE INVENTION

The present invention has been made to solve the aforementioned problems, and it is an object of the present invention to provide a portable electrocardiograph that allows a subject not having thorough medical knowledge to grasp a result of analysis of an electrocardiographic waveform.

A portable electrocardiograph according to one aspect of the present invention includes a storage portion, a plurality of electrodes, a conversion portion, an obtainment portion, a classify portion, a select portion, and a display portion. The storage portion stores combinations of analysis results in association with display contents, the analysis results corresponding to analysis items of an electrocardiographic waveform based on electrocardiography. The electrodes are brought into contact with a subject's body to detect an electric signal for measuring an electrocardiographic waveform of the subject. The conversion portion converts the electric signal detected by the electrodes to a digital signal. The obtainment portion obtains electrocardiographic waveform data of the subject based on the digital signal converted by the conversion portion. The classify portion performs a process for analyzing the electrocardiographic waveform data obtained by the obtainment portion to classify the electrocardiographic waveform data by the analysis items based on electrocardiography. The select portion collates a classification result being classified by the classify portion and the combinations of analysis results stored in the storage portion to select a corresponding display content. The display portion displays the corresponding display content selected by the select portion as an evaluation of the electrocardiographic waveform data.

Desirably, the corresponding display content is prescribed information for informing the subject of the evalution of the electrocardiograph waveform data, indicative of a deviation from a reference electrocardiograph waveform of a normal heart beat.

Desirably, the storage portion further stores urgency levels in association with the combinations of analysis results. In this case, the select portion further selects one of urgency levels corresponding to the classification result, and the display portion further displays the urgency level selected by the select portion.

Further, desirably, the portable electrocardiograph further includes a waveform storage portion and a storage control portion. The waveform storage portion stores the electrocardiographic waveform data obtained by the obtainment portion. A storage control portion controls a process of storing in the waveform storage portion in accordance with the urgency level selected by the select portion.

Further preferably, the storage control portion performs a process of storing the electrocardiographic waveform data in the waveform storage portion when the selected urgency level is a prescribed one, while querying the subject whether or not to store the electrocardiographic waveform data when the selected urgency level is not the prescribed one.

According to the present invention, since an analysis of electrocardiographic waveform data is performed based on electrocardiography, an analysis of high precision can be performed. Further, since the analysis result is displayed as being converted to corresponding display contents, even a subject not having thorough medical knowledge can understand the contents of the analysis result.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic perspective view showing a configuration of a portable electrocardiograph in an embodiment of the present invention.
Fig. 2 is a front view of the portable electrocardiograph shown in Fig. 1.
Fig. 3 is a top view of the portable electrocardiograph shown in Fig. 1.
Fig. 4 is a bottom view of the portable electrocardiograph shown in Fig. 1.
Fig. 5 is a right side view of the portable electrocardiograph shown in Fig. 1.
Fig. 6 is a left side view of the portable electrocardiograph shown in Fig. 1.
Fig. 7 is a perspective view of a measurement posture to be taken by a subject at the time of measuring an electrocardiographic waveform using the portable electrocardiograph in the embodiment of the present invention.
Fig. 8 illustrates a measurement posture to be taken by the subject at the time of measuring an electrocardiographic waveform using the portable electrocardiograph in the embodiment of the present invention, viewed from the above.
Fig. 9 is a functional block diagram of the portable electrocardiograph in the embodiment of the present invention.
Fig. 10 is a flowchart for describing a flow of an electrocardiographic waveform measurement and analysis process with the portable electrocardiograph in the embodiment of the present invention.
Fig. 11 shows exemplary contents of an analysis result correspondence table.
Fig. 12 is a flowchart for describing a flow of an electrocardiographic waveform measurement and analysis process with a portable electrocardiograph in a modification of the embodiment of the present invention.
Fig. 13 shows a typical example of an electrocardiogram.

### DESCRIPTION OF THE PREFERRED EMBODIMENTS

Referring to the drawings, an embodiment of the present invention will be described in detail. Throughout the drawings, the identical or corresponding portions are denoted by an identical reference character, and description thereof is not repeated.

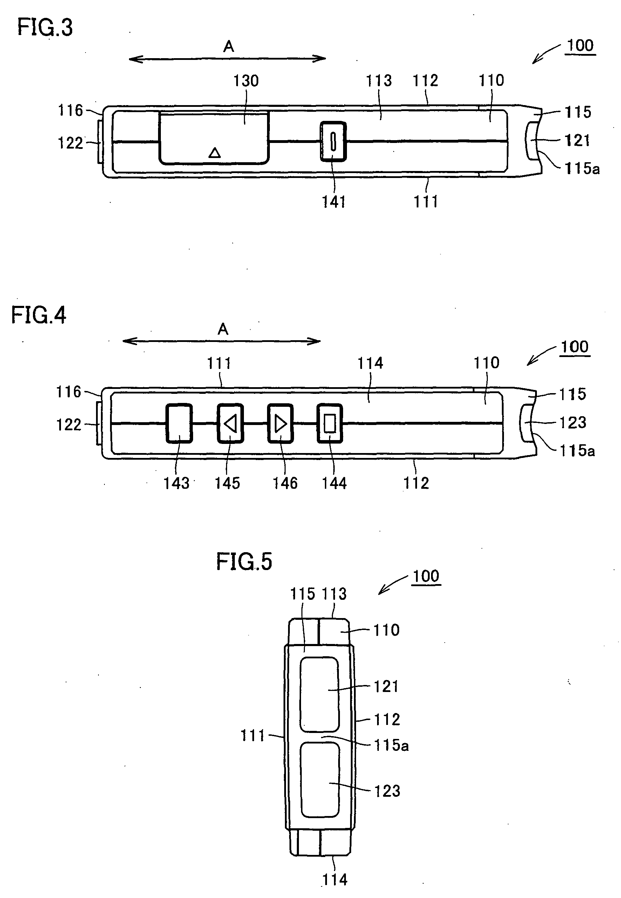

Initially, an appearance of the portable electrocardiograph in the present embodiment will be described. Fig. 1 is a schematic perspective view of a portable electrocardiograph in an embodiment of the present invention. Fig. 2 is a front view of the portable electrocardiograph shown in Fig. 1. Figs. 3 and 4 are respectively top and bottom views of the portable electrocardiograph shown in Fig. 1 when a cover is closed. Figs. 5 and 6 are respectively right side and left side views of the portable electrocardiograph shown in Fig. 1.

As shown in Figs. 1 to 6, in order to realize excellent usability, a portable electrocardiograph 100 in the present embodiment has such a light weight and a small size that it can be held by one hand. Portable electrocardiograph 100 includes a device main body 110 having a flat and elongated, substantially rectangular parallelepiped shape. On its outer surfaces (a front face 111, a rear face 112, a top face 113, a bottom face 114, a right side face 115, and a left side face 116), a display portion, an operation portion, a measurement electrode, and the like are disposed.

As shown in Figs. 1 and 2, a measurement button 142 serving as an operation button for starting measurement is provided in a portion close to one end in the longitudinal direction (the direction shown with an arrow A in the drawing) of front face 111 of device main body 110. In a portion close to the other end of front face 111 of device main body 110, a display portion 148 is provided. Display portion 148 is implemented, for example, by a liquid crystal display and serves to display a result of measurement or the like. The measurement result is displayed, for example, as electrocardiographic waveforms or numerical data as shown in Fig. 1.

As shown in Figs. 1 and 3, a power button 141 is disposed in a prescribed position in top surface 113 of device main body 110. Power button 141 serves as an operation button for turning ON/OFF portable electrocardiograph 100. An opening/closing cover 130, that is the cover, is provided in a prescribed position on top face 113 of device main body 110. Opening/closing Cover 130 is provided in order to cover an external storage medium slot 131 in a cover-closed state, and it is attached to device main body 110 such that it is freely opened and closed.

As shown in Figs. 1 and 4, various operation buttons are disposed in prescribed positions on bottom face 114 of device main body 110. In shown portable electrocardiograph 100, a menu button 143, an enter button 144, a left scroll button 145, and a right scroll button 146 are disposed. Menu button 143 serves as an operation button for displaying a variety of menus for portable electrocardiograph 100, while enter button 144 serves as an operation button to be used for executing a menu and/or each operation. Left scroll button 145 and right scroll button 146 serve as operation buttons to be used for scrolled display of a graph showing a measurement result or guide information displayed on display portion 148.

As shown in Figs. 1 and 5, on right side face 115 located at one end in the longitudinal direction of device main body 110, a negative electrode 121 representing one electrode out of a pair of measurement electrodes as well as an indifferent electrode 123 for deriving a potential serving as a reference in potential variation in the body are disposed. Right side face 115 has a smoothly curved shape, such that a forefinger of the right hand of the subject is fitted thereto when the subject takes a measurement posture which will be described later. In addition, a concave portion 115a extending in an up-down direction is formed in right side face 115. Concave portion 115a is in a shape to receive the forefinger of the right hand of the subject.

Negative electrode 121 and indifferent electrode 123 described above are formed with a conductive member. In addition, negative electrode 121 and indifferent electrode 123 are disposed in concave portion 115a provided in right side face 115 such that their surfaces are exposed on the outer surface of device main body 110. Negative electrode 121 is located closer to top face 113 on right side face 115, while indifferent electrode 123 is located closer to bottom face 114 on right side face 115.

As shown in Fig. 6, on left side face 116 located at the other end in the longitudinal direction of device main body 110, a positive electrode 122 representing the other electrode out of a pair of measurement electrodes is disposed.

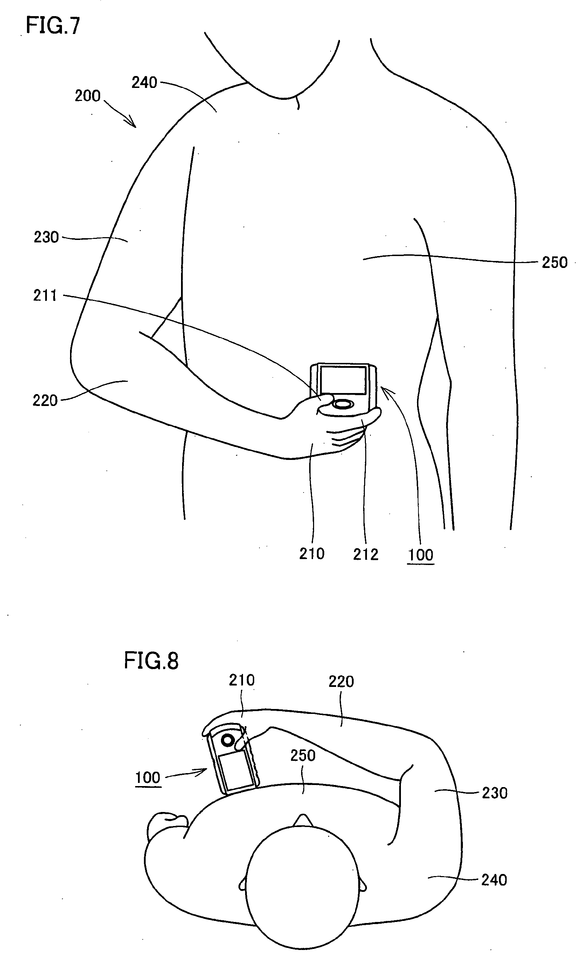

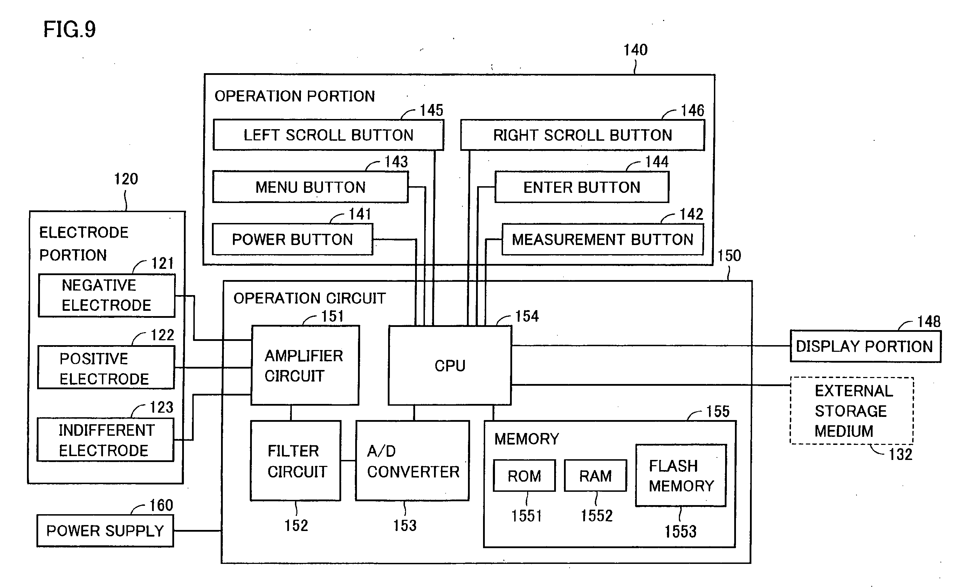

Next, a state where an electrocardiographic waveform is measured using portable electrocardiograph 100 in the embodiment of the present invention will be described. Fig. 7 is a perspective view of a measurement posture to be taken by a subject, while Fig. 8 illustrates the measurement posture viewed from the above.

As shown in Figs. 7 and 8, a subject 200 holds a portion closer to one end of device main body 110 of portable electrocardiograph 100 with his/her right hand 210, and brings positive electrode 122 provided on left side face 116 located at the other end of device main body 110 into contact with the skin on a fifth intercostal anterior axillary line located in a lower left portion of chest 250. Then, the subject presses measurement button 142 provided on front face 111 of device main body 110 with his/her thumb 211 of right hand 210, and maintains this state for several tens of seconds until measurement of the electrocardiographic waveform is completed.

When such a measurement posture is taken, negative electrode 121 and indifferent electrode 123 located on right side face 115 of device main body 110 of portable electrocardiograph 100 come in contact with forefinger 212 of right hand 210 of subject 200, and positive electrode 122 located on left side face 116 of device main body 110 comes in contact with chest 250 of subject 200. In this manner, a measurement circuit is implemented in the body of the subject by right hand 210 being in contact with negative electrode 121, forearm 220 without contacting chest 250, a brachium 230 and a right shoulder 240 without contacting chest 250, and chest 250 to which positive electrode 122 is attached, in this order.

Next, a functional configuration of portable electrocardiograph 100 in the present embodiment is described. Fig. 9 is a functional block diagram of portable electrocardiograph 100 in the embodiment of the present invention.

Referring to Fig. 9, portable electrocardiograph 100 comprises an electrode portion 120 including the aforementioned negative electrode 121, positive electrode 122 and indifferent electrode 123, an operation portion 140 including power button 141, measurement button 142, menu button 143, enter button 144, left scroll button 145, and right scroll button 146, display portion 148, a power supply 160, and a processing circuit 150 that is connected to these components and conducts a variety of processes. Further, process circuit 150 can be connected to an external storage medium 132 which is inserted into external storage medium slot 131.

Processing circuit 150 has an amplifier circuit 151 amplifying a biological electric signal sensed by electrode portion 120, a filter circuit 152 removing a noise component, an A/D (Analog/Digital) converter 153 converting an analog signal to a digital signal, a CPU (central processing portion) 154 performing a variety of operations, and a memory 155. Memory 155 has an ROM (Read Only Memory) 1551, an RAM (Random Access Memory) 1552 and a memory that can store information in a non-volatile manner, e.g., a flash memory 1553.

CPU 154 executes a process of analyzing a digital signal input from A/D converter 153. Further, it receives an instruction signal from various operation buttons included in operation portion 140 and executes a process corresponding to the received instruction signal. Still further, it executes reading and writing of information from/to memory 155. Still further, it exerts display control to display portion 148.

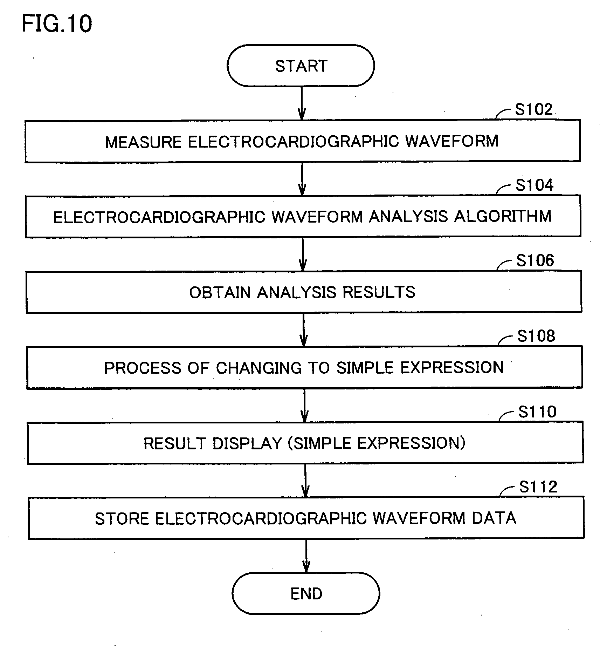

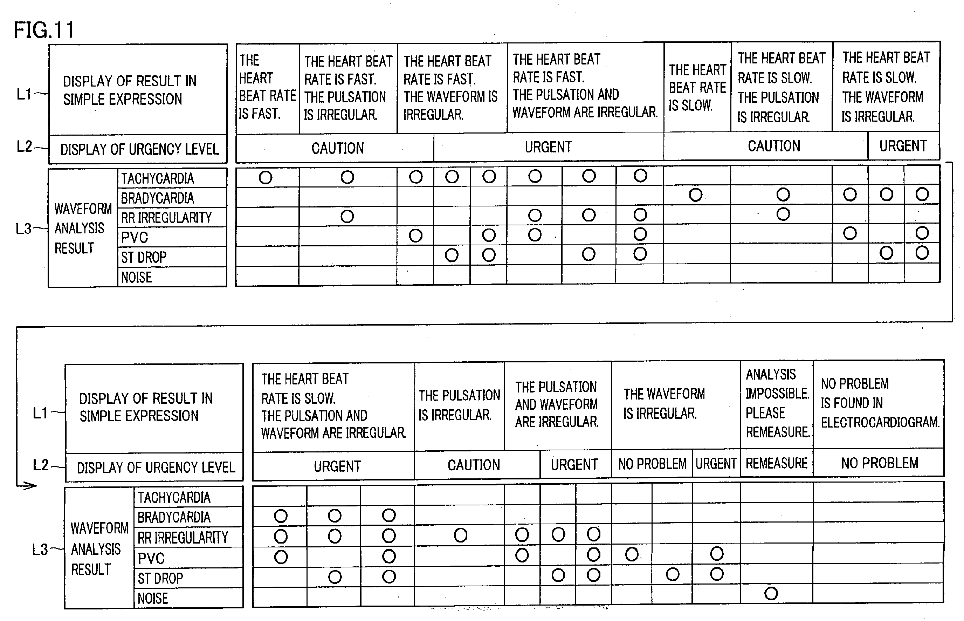

Next, referring to Figs. 10 and 11, an electrocardiographic waveform measurement and analysis process with portable electrocardiograph 100 is described.

Fig. 10 is a flowchart for describing the flow of an electrocardiographic waveform measurement and analysis process with portable electrocardiograph 100 of the embodiment of the present invention.

Initially, when an instruction of starting electrocardiographic waveform measurement is input by pressing-down of measurement button 142, CPU 154 reads a measurement program stored in ROM 1551 and starts to measure an electrocardiographic waveform of a subject (step S 102).

An electric signal detected by electrode portion 120 is amplified by amplifier circuit 151, from which a noise is removed by filter circuit 152. Then, the electric signal from which a noise is removed is converted to a digital signal by A/D converter 153. CPU 154 obtains the digital signal converted by A/D converter 153, and thereby obtains electrocardiographic waveform data of the subject. It displays the obtained electrocardiographic waveform data on display portion 148 in real time, and temporarily stores the data in RAM 1552.

Such a process is performed until a prescribed time is measured by a timer (not shown), for example.

When the measurement of the electrocardiographic waveform ends, CPU 154 analyses the electrocardiographic waveform data having been stored in RAM 1552 by an electrocardiographic waveform analysis algorithm based on electrocardiography (step S104). Specifically, by classifying the obtained electrocardiographic waveform data by analysis items based on electrocardiography, the waveform analysis is performed.

Here, "analysis items based on electrocardiography" refers to prescribed analysis items obtained by detecting a deviation from a reference electrocardiographic waveform of a normal heart beat, for example by an analysis algorithm shown in Prior Art Document 4. This "reference electrocardiographic waveform of a normal heart beat" (hereinafter abbreviated as "normal waveform") refers to an electrocardiographic waveform that attains a reference value (a normal value) of the subject that is determined in advance for example by the clustering method described in Prior Art Document 4 or the like.

Further, "prescribed analysis items" represent in the present embodiment five items of "tachycardia" "bradycardia" "RR irregularity" "PVC (Premature Ventricular Contraction)", and "ST drop", as well as the sixth item "noise". Accordingly, in the present embodiment, the analysis items based on electrocardiography represent these six items. It is noted that the analysis items are not limited thereto, and for example, other items may be added.

Here, the six analysis items are briefly described. In general, "tachycardia" refers to a heart rate of at least 100 beats per minute, while "bradycardia" refers to a heart rate of at most 50 beats per minute. "RR irregularity" refers to R-R intervals of QRS wave being irregular, and for example, refers to the rhythm of QRS wave being irregular, skipping one beat and the like. "PVC" refers to arrhythmia originating in the ventricles of heart, in which pulsation different from normal pulsation occurs. As its characteristic electrocardiographic waveform, a broad QRS wave without P wave is detected. "ST drop" refers to ST portion between QRS wave and T wave being dropped, which suggests a possibility of ischemic heart disease. "Noise" may be generated by baseline fluctuations, for example.

More specifically, the process of electrocardiographic waveform analysis at the aforementioned step S 104 is performed by CPU 154 detecting, from the obtained electrocardiographic waveform data, presence/absence of characteristics in shape indicative of arrhythmia or ischemia of cardiac muscle, presence/absence of characteristics in cycle indicative of tachycardia or bradycardia, presence/absence of a waveform that cannot be analyzed due to noise or baseline fluctuations. CPU 154 analyses a result of the detection, and classifies the electrocardiographic waveform data by the six analysis items. When the data is applicable to any of the analysis items in this classification process, CPU 154 sets flags in the applicable fields of a classification table by the six analysis items developed in RAM 1552, for example.

As described above, since an automatic analysis is performed using an analysis algorithm that is premised to be used by medical specialists in a clinic, as shown in Prior Art Document 4, an analysis result of high precision can be obtained.

Then, when the waveform analysis by the electrocardiographic waveform analysis algorithm at step S104 ends, CPU 154 obtains results of the analysis (step S106). For example, it obtains the results of the analysis by reading analysis items to which a flag/flags is/are set in the classification table in RAM 1552.

Next, CPU 154 refers to an analysis result correspondence table stored in ROM 1551 in advance, and thereby performs a process of changing to simple expression (step S108).

Now, the analysis result correspondence table is described. Fig. 11 shows exemplary contents of an analysis result correspondence table. Referring to Fig. 11, in the region denoted by L3 in the analysis result correspondence table, combinations of analysis results by six analysis items based on electrocardiography is stored. The combinations of analysis results are represented by "o" allotted to rows of respective analysis items.

In the region denoted by L1, data of display of result in simple expression is stored, while in the region denoted by L2, data of display of urgency level is stored.

The data of display of result in simple expression stored in region L1 is prescribed information for informing the subject of evalution of an electrocardiographic waveform, indicative of a deviation from a normal waveform. Specifically, it is information indicative of a heart beat rate, an extent of irregularity of heart pulsation and electrocardiographic waveform and the like. It is noted that the "simple expression" refers to an expression that is different from diagnostic terms for electrocardiographic waveforms used by doctors, and that can be understood by a general subject. In the present embodiment, for example, expression by text may be used.

The data of display of urgency level stored in region L2 indicates an extent of recommendation to have a doctor's diagnosis. In the present embodiment, for example, any of four items of "urgent", "caution", "no problem", and "remeasure" is indicated. "Urgent" means that it is strongly recommended to have a doctor's diagnosis, while "caution" means that it is better to have a doctor's diagnosis. "No problem" means that it is not specifically necessary to have a doctor's diagnosis, while "remeasure" means that remeasurement is simply requested without relation to a doctor's diagnosis.

Fig. 11 shows that the data of display is stored in respective regions L1 and L2 of a column corresponding to each combination of analysis results of region L3. In the following, when simply described as "data of display", both data of display of result in simple expression stored in region L1 and data of display of urgency level stored in region L2 are included.

It is noted that, in the present embodiment, only the data of display of result in simple expression shown in region L1 may be stored in correspondence with each combination of analysis results of region L3. In such a case, "data of display" does not include the data of display of urgency level stored in region L2.

Referring to Fig. 11, for example, when an analysis result shown in region L3 is applicable only to "tachycardia", referring to the relevant column, it is shown that the corresponding data of display of result in simple expression is "The heart rate is fast." (region L1), while the corresponding data of display of urgency is "caution" (region L2). When a combination of analysis results shown in region L3 is applicable to the three items of "bradycardia", "RR irregularity" and "PVC", referring to the relevant column, it is shown that the corresponding data of display of result in simple expression is "The heart rate is slow. The pulsation and waveform are irregular." (region L1), while the corresponding data of display of urgency is "urgent" (region L2).

When an analysis result shown in region L3 is applicable to "noise", referring to the relevant column, it is shown that the corresponding data of display of result in simple expression is "Analysis impossible. Please remeasure." (region L1), while the corresponding data of display of urgency is "remeasure" (region L2). Further, when there is no applicable analysis result, referring to a column without o mark in region L3, it is shown that the corresponding data of display of result in simple expression is "No problem is found in electrocardiogram." (region L1), while the corresponding data of display of urgency is "No problem" (region L2).

Now, the data of display predetermined as above is described in further detail.

The data of display of result in simple expression in region L1 has the following relationship. Among the analysis items, the simple expression corresponding to "tachycardia" and "bradycardia" relates to a heart beat rate. "RR irregularity" relates to irregularity of pulsation. "PVC and "ST drop" relate to irregularity of electrocardiographic waveform.

The data of display of urgency level in region L2 has the following relationship. Among the analysis items, high urgency level is set to "ST drop", since there is a possibility of ischemic heart disease. High urgency level is set to "PVC" when it appears with "RR irregularity", "bradycardia" or "tachycardia", since there is a possibility of critical arrhythmia such as atrial fibrillation, ventricular fibrillation or atrioventricular block.

It is noted that, although "simple expression" in region L1 is expression by text in the present embodiment, it is not limited thereto. For example, a heart beat rate and an extent of irregularity of pulsation of heart and electrocardiographic waveform may be expressed in symbols, level expressions or the like. If past analysis results of electrocardiographic waveform is stored in flash memory 1553, for example, then the expression may be a comparison against the past analysis results. In such a case, the data of display of result in simple expression may be "The heart beat rate is increased. ", "While the heart beat rate becomes normal, the waveform is still irregular. ", or the like.

The data of display of urgency level in region L2 may be data of colors such as red, blue, green and the like. It may also be suggestions of the next action to be taken, such as "Go to a hospital immediately. ", "Take a deep breath slowly and rest." or the like.

Referring again to Fig. 10, at step S108, CPU 154 collates the analysis results of electrocardiographic waveform obtained at step S106 and combinations of analysis results stored in region L3 of the analysis result correspondence table. Then, it selects data of display corresponding to the matched combination of analysis results.

Specifically, it selects data of display in a column where a combination of the obtained analysis results is stored in region L3 of the analysis result correspondence table shown in Fig. 11. Thus, the analysis results of the electrocardiographic waveform are changed to simple expression represented by the selected data of display.

For example, referring to Fig. 11, when a combination of analysis results applies to the aforementioned three of "bradycardia", "RR irregularity", and "PVC", then data of display of result in simple expression of "The heart rate is slow. The pulsation and waveform are irregular" (region L1) and data of display of urgency level of "urgent" (region L2) are selected.

When a series of analysis process steps of the aforementioned steps S104-S108 ends, CPU 154 performs result display on display portion 148 (step S110). At step S 110, CPU 154 displays the data of display selected at step S108 as evaluation of the electrocardiographic waveform. Accordingly, in the above-described example, display 148 displays "The heart rate is slow. The pulsation and waveform are irregular." and "urgent".

As the subject is informed in the simple expressions, even a subject without thorough medical knowledge can grasp the evaluation of an electrocardiographic waveform. Additionally, as the evaluation informed to the subject corresponds to the results obtained from automatic analysis using an analysis algorithm used by medical specialists, an optimum evaluation can be provided to the subject.

Next, after performing the result display at step S 110, CPU 154 performs a process of storing in flash memory 1553 the electrocardiographic waveform data having been temporarily stored in RAM 1552 and the analysis results thereof (step S 112). By storing the electrocardiographic waveform data and the analysis results, the subject can have a doctor's diagnosis later based on the data. Thus, the electrocardiographic waveform measurement and analysis process ends.

It is noted that the process of storing in flash memory 1553 at step S 112 may not necessarily follow step S 110, and it may be performed between steps S 108 and S 110, for example. Additionally, at step S 112, for example a query as to whether or not to store electrocardiographic waveform data and the like may be displayed on display portion 148, and the electrocardiographic waveform data and the analysis results may be stored in flash memory 1553 in accordance with an instruction signal from operation portion 140.

The electrocardiographic waveform measurement process at step S 102 and the electrocardiographic waveform data analyze process at step S104 may be performed in parallel.

### Modification

Next, a portable electrocardiograph 100 in a modification of the embodiment of the present invention will be described.

Portable electrocardiograph 100 in the modification of the embodiment of the present invention is different in the method related to a electrocardiographic waveform data storing process (step S 112 in Fig. 10) in the electrocardiographic waveform measurement/analysis process of the above-described embodiment. Since the configuration and basic operation of portable electrocardiograph 100 is the same as in the above-described embodiment, description thereof is not repeated.

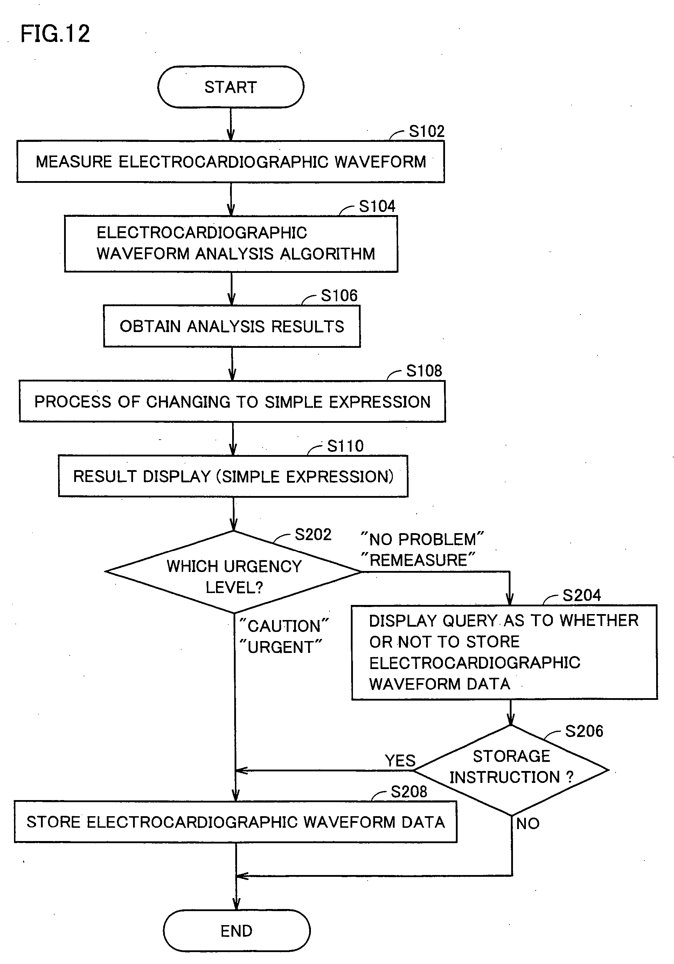

Fig. 12 is a flowchart for describing the flow of an electrocardiographic waveform measurement and analysis process with portable electrocardiograph 100 in the modification of the embodiment of the present invention. Since the process steps at step S102-S110 are the same as those shown in the flowchart of Fig. 10, description there of is not repeated.

In contrast to the above-described embodiment where electrocardiographic waveform data is automatically saved at step S 112, in the modification, CPU 154 determines "urgency level" at step S202.

When data of display of urgency level selected at step S 108 is "urgent" or "caution", CPU 154 stores in flash memory 1553 the electrocardiographic waveform data having been temporarily stored in RAM 1552 and the analysis results thereof (step S208).

On the other hand, when data of display of urgency level selected at step S108 is "no problem" or "remeasure", CPU 154 displays on display portion 148 a query as to whether or not to store electrocardiographic waveform data and the like (step S204). Then, when it senses from an instruction signal from operation portion 140 that a storage instruction is provided from the subject (YES at step S206), it stores in flash memory 1553 the electrocardiographic waveform data having been temporarily stored in RAM 1552 and the analysis results thereof (step S208). On the other hand, when an storage instruction is not provided from the subject (NO at step S206), it does not store the data in flash memory 1553 and ends the process.

The process steps of steps S202-S208 may be performed between steps S108 and S110.

According to the modification of the embodiment of the present invention, in a case of high urgency level, that is, when it is recommended to have a doctor's diagnosis according to the results, the electrocardiographic waveform data and the analysis results thereof are saved automatically. Accordingly, if the analysis results indicates the necessity to have a doctor's diagnosis, the electrocardiographic waveform data can surely be saved. Thus, the subject will not fail to save the electrocardiographic waveform data of high urgency level.

When the urgency level is low, that is, when it is not specifically necessary to have a doctor's diagnosis according to the results, the data can be saved arbitrarily by the subject. Accordingly, the electrocardiographic waveform data of low urgency level will not be saved without reason. Thus, the subject can have a doctor diagnose only electrocardiographic waveform data of high urgency.

It is noted that, in the present modification of the embodiment, the urgency level may only be employed in the determination at step S202. Specifically, at step S110, the urgency level may not be displayed.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A portable electrocardiograph, comprising:
a storage portion (1551) storing combinations of analysis results in association with display contents, said analysis results corresponding to analysis items of an electrocardiographic waveform based on electrocardiography;
a plurality of electrodes (120) brought into contact with a subject's body to detect an electric signal for measuring an electrocardiographic waveform of said subject;
a conversion portion (153) converting the electric signal detected by said electrodes to a digital signal;
an obtainment portion (S102) obtaining electrocardiographic waveform data of said subject based on the digital signal converted by said conversion portion;
a classify portion (S104) performing a process for analyzing said electrocardiographic waveform data obtained by said obtainment portion to classify said electrocardiographic waveform data by said analysis items based on electrocardiography;
a select portion (S108) collating a classification result being classified by said classify portion and said combinations of analysis results stored in said storage portion to select a corresponding display content; and
a display portion (148) displaying said corresponding display content selected by said select portion as an evaluation of said electrocardiographic waveform data.

2. The portable electrocardiograph according to claim 1, wherein
said corresponding display content is prescribed information for informing the subject of said evalution of said electrocardiograph waveform data, indicative of a deviation from a reference electrocardiograph waveform of a normal heart beat.

3. The portable electrocardiograph according to claim 1, wherein
said storage portion further stores urgency levels in association with said combinations of analysis results,
said select portion further selects one of said urgency levels corresponding to said classification result, and
said display portion further displays said urgency level selected by said select portion.

4. The portable electrocardiograph according to claim 3, further comprising:
a waveform storage portion (1553) storing said electrocardiographic waveform data obtained by said obtainment portion; and
a storage control portion (S208) controlling a process of storing in said waveform storage portion in accordance with said urgency level selected by said select portion.

5. The portable electrocardiograph according to claim 4, wherein
said storage control portion performs a process of storing said electrocardiographic waveform data in said waveform storage portion when said selected urgency level is a prescribed one, while querying said subject whether or not to store said electrocardiographic waveform data when said selected urgency level is not said prescribed one.
